# EUROPEAN PATENT APPLICATION

(11) **EP 4 198 122 A1**
(43) Date of publication of application: **21.06.2023**
(21) Application number: 21857662.7
(22) Date of filing: 17.08.2021
(51) Int. Cl.: C12N 5/0793, A61K 38/18, C12N 5/0797

(54) **EXPANSION CULTURE MEDIUM AND CULTURE METHOD FOR NEURAL CELLS**

(30) Priority: 17.08.2020 CN 202010824749
(71) Applicant: Institute Of Zoology, Chinese Academy Of Sciences, Beijing 100101 (CN); Beijing Institute for Stem Cell and Regenerative Medicine, Beijing 100101 (CN)
(72) Inventor: ZHOU, Qi, Beijing 100101 (CN); LI, Wei, Beijing 100101 (CN); HAO, Jie, Beijing 100190 (CN); WANG, Yukai, Beijing 100190 (CN); FENG, Lin, Beijing 100190 (CN); WANG, Liu, Beijing 100101 (CN); HU, Baoyang, Beijing 100101 (CN); SUN, Yun, Beijing 100101 (CN); TIAN, Yao, Beijing 100101 (CN); HE, Zhengquan, Beijing 100101 (CN)
(74) Representative: Bottero, Carlo
(86) International application number: PCT/CN2021/112986
(87) International publication number: WO 2022/037570

(57) **Abstract**

A culture medium and a culture method for neural cells, particularly relating to a composition comprises a SMAD signaling pathway inhibitor, a SHH signaling pathway agonist, a Wnt signaling pathway agonist and a Myosin II ATPase inhibitor, optionally, it further comprises a ROCK inhibitor; or, the composition consists of the SMAD signaling pathway inhibitor, the SHH signaling pathway agonist, the Wnt signaling pathway agonist, the Myosin II ATPase inhibitor, and optionally the ROCK inhibitor. The same batch of neural cells, such as midbrain dopaminergic progenitors, can be efficiently obtained by using the culture method and culture medium designed on the basis of the composition.

## Description

The present application is based on the application with CN application number of 202010824749.3 and the application date of August 17, 2020, and claims its priority. The disclosure content of this CN application is hereby incorporated into the present application in its entirety.

### Technical Field

The present application relates to nerve cell culture technology, in particular to an expansion culture medium and culture method for nerve cells. A large number of nerve cells can be efficiently obtained by using the culture medium and culture method, and the nerve cells having stable quality can be obtained in subsequent differentiation, thereby realizing cell therapy for diseases caused by nerve cell defects (e.g., Parkinson's disease).

### Background Art

Parkinson's disease is a degenerative disease of the central nervous system that commonly occurs in middle-aged and elderly people. The main pathogenesis is the massive death of midbrain dopaminergic neurons in the substantia nigra, resulting in a decrease in the synthesis of dopamine transmitter in the brain, thereby affecting behavior. The midbrain dopaminergic neurons in the substantia nigra are differentiated from the midbrain floor plate cells in the embryonic period, and project to the striatum to form a loop. They release dopamine transmitter after maturation and play an important role in regulating individual movements.

The midbrain floor plate cells can be isolated from early embryos, and can also be obtained by in vitro differentiation from pluripotent stem cells. Existing differentiation methods simulate the signaling pathways of early embryonic development in vivo, using BMP and TGF inhibitors to double inhibit SMAD signaling pathways and activating Wnt signaling and SHH signaling so as to specialize pluripotent stem cells into midbrain floor plate cells. Then, under the treatment of FGF8, DAPT, BDNF, GDNF, etc., the midbrain floor plate cells are further differentiated into dopaminergic neurons.

Midbrain dopaminergic neurons are a specific subpopulation of neurons. During the culture process, a small amount of Wnt signal and a high dose of SHH are required in the progenitor stage, and then FGF8 needs to be added to promote maturation. Compared with other cells, the culture conditions are very unique. At present, the problems existing in the culture process are mainly manifested in: 1) the quality of midbrain floor plate cells differentiated from pluripotent stem cells in vitro is uneven; 2) the stability of differentiation batches is poor; 3) the production of midbrain dopaminergic nerve cells lacks a stable bulk expansion process for intermediate cells.

In addition, other nerve cells or nerve-related cells such as GABA progenitor cells, astrocytes, and microglial cells also play an important role in Parkinson's disease, but they also face the problem of unstable expansion.

### Contents of the Invention

After a lot of creative work, the inventors found that by adjusting the composition of the medium and adding the Myosin II ATPase inhibitor Blebbistatin, the expansion efficiency of nerve cells or nerve-related cells such as midbrain dopaminergic progenitors, GABA progenitors, astrocytes and microglial cells can be significantly increased, and the high-quality cells can be selected for batch expansion, which provides a stable way to obtain high-quality nerve cells or nerve-related cells (e.g., dopaminergic neurons, GABA progenitors, astrocytes, microglial cells, etc.), laying the foundation for cell therapy for Parkinson's and other diseases caused by dopaminergic cell defects.

In the first aspect, the present application provides a composition, which comprises one or more additives for maintaining and/or improving the activity and/or function of nerve cells, and a Myosin II ATPase inhibitor.

In some embodiments, the composition comprises a SMAD signaling pathway inhibitor, a SHH signaling pathway agonist, a Wnt signaling pathway agonist and a Myosin II ATPase inhibitor, optionally, further comprises a ROCK inhibitor;
alternatively, the composition consists of a SMAD signaling pathway inhibitor, a SHH signaling pathway agonist, a Wnt signaling pathway agonist, a Myosin II ATPase inhibitor, and optionally a ROCK inhibitor.

In an embodiment of the present application, the Myosin II ATPase inhibitor refers to a myosin type II ATPase inhibitor. In certain embodiments, the Myosin II ATPase inhibitor is selected from Blebbistatin and a derivative thereof, for example, Blebbistatin, namely (S)-(-)-Blebbistatin, having a structure of 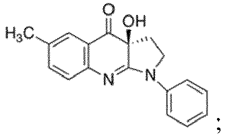 for example, (S)-(-)-Blebbistatin O-Benzoate, having a structure of

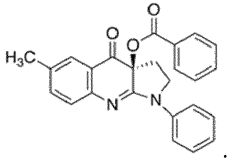

In certain embodiments, the SMAD signaling pathway inhibitor is selected from a BMP inhibitor, a TGFβ/Activin-Nodal inhibitor and a combination thereof.

In an embodiment of the present application, the BMP (bone morphogenetic protein) inhibitor is an inhibitor of BMP signaling pathway. In certain embodiments, the BMP inhibitor is selected from the group consisting of DMH-1, Dorsomorphin, Noggin, LDN193189, and any combination thereof. Among them, LDN193189 refers to the small molecule DM-3189, its chemical formula is C₂₅H₂₂N₆, and its chemical name is 4-(6-(4-(piperidin-1-yl)phenyl)-pyrazolo[1,5-a] pyrimidin-3-yl)quinoline. It acts as an inhibitor of SMAD signaling pathway, and is also a potent small molecule inhibitor of ALK2, ALK3, ALK6, and PTK. It can inhibit the signaling pathway of TGFβ1 receptor ALK1 and ALK3 family members, resulting in the inhibition of multiple biological signal transduction, including BMP (e.g., BMP2, BMP4, BMP6, BMP7) and Activin, and the subsequent phosphorylation of SMAD (e.g., Smad1, Smad5 and Smad8).

In an embodiment of the present application, the TGFβ/Activin-Nodal inhibitor is a substance that hinders the pathway from the binding of TGFβ with its receptor to continuously transmit signals to SMAD, which can be selected from a substance that hinders the binding with the ALK family as a receptor, or a substance that hinders the phosphorylation of SMAD caused by ALK family. In certain embodiments, the TGFβ/Activin-Nodal inhibitor is selected from the group consisting of SB431542, SB505124, A83-01, and any combination thereof. Among them, SB431542 refers to a small molecule that can reduce or block the transcription of TGF/Activin-Nodal signaling pathway, which has the CAS number 301836-41-9, molecular formula C₂₂H₁₈N₄O₃, and chemical name 4-[4-(1,3-benzodioxan-5-yl)-5-(2-pyridyl)-1H-imidazol-2-yl]-benzamide.

In an embodiment of the present application, the SHH signaling pathway agonist refers to a substance that causes SHH to bind to Patched (Ptch1) as a receptor, resulting in de-inhibition of Smoothenod (Smo) and further causing Gli2 activation. In certain embodiments, the SHH signaling pathway agonist is selected from a group consisting of a SHH protein, a Smoothend agonist and a combination thereof. In certain embodiments, the SHH protein is selected from the group consisting of recombinant SHH and terminal-modified SHH (e.g., SHH C25II). In certain embodiments, the Smoothend agonist is selected from the group consisting of SAG, Hh-Ag1.5, 20α-hydroxycholesterol, Purmorphamine, and any combination thereof. SAG has the molecular formula C₂₈H₂₈ClN₃OS, and the CAS number 912545-86-9. Purmorphamine is a purine derivative with CAS number 483367-10-8, and can activate Hedgehog signaling pathway including by targeting Smoothened.

In an embodiment of the present application, the Wnt signaling pathway refers to a signaling pathway composed of a Wnt family ligand and a Wnt family receptor. In certain embodiments, the Wnt signaling pathway agonist is selected from the group consisting of a GSK3β inhibitor, Wnt3A, Wnt1 and a combination thereof.

In an embodiment of the present application, the GSK3β inhibitor refers to a compound that inhibits glycogen synthase kinase 3β. The GSK3β inhibitor in the present application can activate the Wnt signaling pathway. In certain embodiments, the GSK3β inhibitor is selected from the group consisting of CHIR99021, GSK3β inhibitor IX (6-bromoindirubin-3'-oxime, BIO), GSK3β inhibitor VII (4-dibromoacetophenone), Indirubin, L803-mts, TWS119, AZD2858, AR-A014418, TDZD-8, LY2090314, 2-D08, IM-12, 1-Azakenpaullone, SB216763, or any combination thereof. Among them, CHIR99021 refers to 6-(2-(4-(2,4-dichlorophenyl)-5-(4-methyl-1H-imidazol-2-yl) pyrimidine-2-amino)ethylamino)nicotinonitrile. It is a small molecule inhibitor of GSK3β, and can activate Wnt signaling pathway.

In an embodiment of the present application, the ROCK inhibitor is a substance that inhibits the function of Rho kinase (ROCK), such as Y-27632, HA100 or HA1152. In certain embodiments, the ROCK inhibitor is Y-27632.

In some embodiments, the composition comprises a BMP inhibitor, a TGFβ/Activin-Nodal inhibitor, a SHH protein, a Smoothened agonist, a GSK3β inhibitor, a Myosin II ATPase inhibitor, and optionally a ROCK inhibitor;
alternatively, the composition consists of a BMP inhibitor, a TGFβ/Activin-Nodal inhibitor, a SHH protein, a Smoothened agonist, a GSK3β inhibitor, a Myosin II ATPase inhibitor, and optionally a ROCK inhibitor.

In certain embodiments, the composition comprises LDN193189, SB431542, SHH, SAG, CHIR99021, Blebbistatin, and optionally Y-27632;
alternatively, the composition consists of LDN193189, SB431542, SHH, SAG, CHIR99021, Blebbistatin and optionally Y-27632.

In certain embodiments, the composition comprises a N2 supplement, a B27 supplement, bFGF (recombinant basic fibroblast growth factor) and Blebbistatin;
alternatively, the composition consists of a N2 supplement, a B27 supplement, bFGF and Blebbistatin.

In a second aspect, the present application provides a culture medium, which comprises the aforementioned composition, and a basal culture medium.

In certain embodiments, the basal culture medium is suitable for the cultivation of a nerve cell.

In some embodiments, the basal culture medium is selected from the group consisting of N2 medium, DMEM medium, DMEM/F12 medium, Neurobasal culture medium or/and Sciencell 1801 medium.

In certain embodiments, the basal culture medium is N2 medium.

In certain embodiments, the basal culture medium is a basal culture medium supplemented with the following substances: one or more serum-free substitutes, glutamine or a stabilized dipeptide of L-alanyl-L-glutamine.

In certain embodiments, the basal culture medium is a basal culture medium supplemented with the following substances: a N2 supplement and a stabilized dipeptide of L-alanyl-L-glutamine.

In certain embodiments, the basal culture medium is composed of the follows: 49% CTS^{™} KnockOut^{™} DMEM/F-12 + 49% CTS^{™} Neurobasal + 1% CTS^{™} N2 Supplement + 1% CTS-GlutaMAX^{™}-I.

In certain embodiments, the components of the N2 medium is as follows: 49% KODMEM + 49% Neurobasal + 1% N2-supplement + 1% GlutaMAX.

In some embodiments, the concentration of each component in the medium is an effective concentration for realizing the respective biological function thereof.

In some embodiments, the concentration of the BMP inhibitor can be, for example, 0.05-1 µM, but not limited thereto. In some specific embodiments, the concentration of the BMP inhibitor (e.g., LDN193189) can be 50 nM, 60 nM, 70 nM, 80 nM, 90 nM, 100 nM, 200 nM, 300 nM, 400 nM, 500 nM, 600 nM, 700 nM, 800 nM, 900 nM, or 1000 nM. The preferred concentration is 100 nM.

In certain embodiments, the concentration of the TGFβ/Activin-Nodal inhibitor can be 5-20 µM, but not limited thereto. In some specific embodiments, the concentration of the TGFβ/Activin-Nodal inhibitor (e.g., SB431542) can be 5 µM, 6 µM, 7 µM, 8 µM, 9 µM, 10 µM, 11 µM, 12 µM, 13 µM, 14 µM, 15 µM, 16 µM, 17 µM, 18 µM, 19 µM, or 20 µM. The preferred concentration is 10 µM.

In certain embodiments, the concentration of the SHH protein can be 50-200 ng/mL, but not limited thereto. In some specific embodiments, the concentration of the SHH protein (e.g., recombinant SHH) is, for example, 50 ng/mL, 60 ng/mL, 70 ng/mL, 80 ng/mL, 90 ng/mL, 100 ng/mL, 110 ng/mL, 120 ng/mL, 130 ng/mL, 140 ng/mL, 150 ng/mL, 160 ng/mL, 170 ng/mL, 180 ng/mL, 190 ng/mL, or 200 ng/mL. The preferred concentration is 100 ng/mL.

In certain embodiments, the concentration of the smoothened agonist can be 0.5-3 µM, but not limited thereto. In some specific embodiments, the concentration of the Smoothened agonist (e.g., SAG) is 0.5 µM, 0.6 µM, 0.7 µM, 0.8 µM, 0.9 µM, 1.0 µM, 1.2 µM, 1.4 µM, 1.6 µM, 1.8 µM, 2.0 µM, 2.5 µM, or 3.0 µM. The preferred concentration is 2.0 µM.

In certain embodiments, the concentration of the GSK3β inhibitor can be 0.5-1.0 µM, but not limited thereto. In some specific embodiments, the concentration of the GSK3β inhibitor (e.g., CHIR99021) is 0.5 µM, 0.6 µM, 0.7 µM, 0.8 µM, 0.9 µM or 1.0 µM.

In certain embodiments, the concentration of the Myosin II ATPase inhibitor can be 5-20 µM, but not limited thereto. In some specific embodiments, the concentration of the Myosin II ATPase inhibitor (e.g., Blebbistatin) is 5 µM, 6 µM, 7 µM, 8 µM, 9 µM, 10 µM, 11 µM, 12 µM, 13 µM, 14 µM, 15 µM, 16 µM, 17 µM, 18 µM, 19 µM or 20 µM. The preferred concentration is 10 µM.

In certain embodiments, the concentration of the ROCK inhibitor can be 1-50 µM, but not limited thereto. In certain embodiments, the concentration of the ROCK inhibitor is 1-30 µM, such as 5-30 µM. In some specific embodiments, the concentration of the ROCK inhibitor (e.g., Y-27632) is 1 µM, 2 µM, 3 µM, 4 µM, 5 µM, 6 µM, 7 µM, 8 µM, 9 µM, 10 µM, 11 µM, 12 µM, 13 µM, 14 µM, 15 µM, 16 µM, 17 µM, 18 µM, 19 µM, 20 µM, 21 µM, 22 µM, 23 µM, 24 µM, 25 µM, 26 µM, 27 µM, 28 µM, 29 µM, or 30 µM. The preferred concentration is 10 µM.

In some embodiments, the medium comprises a N2 medium, 10 µM SB431542, 100 nM LDN193189, 100 ng/mL SHH, 2 µM SAG, 0.5-1.0 µM CHIR99021, and 10 µM Blebbistatin.

In some embodiments, the culture medium is suitable for culturing, for example expanding, a midbrain dopamine neuron progenitor.

In certain embodiments, the basal culture medium is DMEM/F12 and Neurobasal.

In some embodiments, the medium comprises 49.25% DMEM/F12, 49.25% Neurobasal, 0.5% N2 supplement, 1% B27 supplement, 10 ng/ml bFGF (recombinant basic fibroblast growth factor) and 5 -20 µM Blebbistatin.

In certain embodiments, the medium is suitable for culturing, for example expanding, a GABAergic neuronal progenitor.

In some embodiments, the basal culture medium is NIM (Neural Induction Media).

In some embodiments, the culture medium comprises DMEM/F12, Neurobasal, N2 supplement, B27 supplement and Glutamax10, EGF, FGF2, TGFb1 and 5-20 µM Blebbistatin.

In certain embodiments, the medium is suitable for culturing, for example expanding, an astrocyte.

In some embodiments, the basal culture medium is DMEM/F12, KOSR, 1×Glutamax, 1X NEAA, BMP4, VEGF, SCF, X-VIVO, IL-34, Y-27632 and 5-20 µM Blebbistatin.

In certain embodiments, the culture medium is suitable for culturing, for example expanding, a microglial cell.

In some embodiments, the medium comprises 49.25% DMEM/F12, 49.25% Neurobasal, 0.5% N2 supplement, 1% B27 supplement, 10 ng/ml bFGF (recombinant basic fibroblast growth factor) and 5 -20 µM Blebbistatin.

In certain embodiments, the medium is suitable for culturing, for example expanding, a GABAergic neuronal progenitor.

In certain embodiments, the basal culture medium is Sciencell 1801.

In certain embodiments, the medium comprises 5-20 µM Blebbistatin.

In certain embodiments, the culture medium is suitable for culturing, for example expanding, a glial cell.

In certain embodiments, the medium is a basal culture medium supplemented with the following substances: one or more serum-free substitutes, glutamine or a stabilized dipeptide of L-alanyl-L-glutamine, EGF, FGF2, TGFβ1, and Blebbistatin or a derivative thereof. In certain embodiments, the basal culture medium is a basal culture medium supplemented with the following substances: a N2 supplement, a B27 supplement, a stabilized dipeptide of L-alanyl-L-glutamine, EGF, FGF2, TGFβ1 and Blebbistatin. In certain embodiments, the medium is composed of the follows: 48% DMEM/F-12 + 48% Neurobasal + 1% N2 + 2% B27 + 1% GlutaMAX + 10ng/ml EGF + 10ng/ml FGF2 + 10ng/ml TGFβ1 + 10 µM Blebbistatin. In some embodiments, the medium is particularly suitable for the expansion culture of an astrocyte.

In certain embodiments, the medium is a basal culture medium supplemented with the following substances: one or more serum-free substitutes, glutamine or a stabilized dipeptide of L-alanyl-L-glutamine, a SHH signaling pathway inhibitor, and Blebbistatin or a derivative thereof. In certain embodiments, the basal culture medium is a basal culture medium supplemented with the following substances: a N2 supplement, a B27 supplement, a stabilized dipeptide of L-alanyl-L-glutamine, SHH, Purmorphamine, and Blebbistatin. In certain embodiments, the medium is composed of the follows: 48% DMEM/F-12 + 48% Neurobasal + 1% N2 + 2% B27 + 1% GlutaMAX + 100 ng/mL SHH + 1 µM purmorphamine + 10 µM Blebbistatin. In certain embodiments, the medium is particularly suitable for the expansion culture of a GABAergic neural progenitor cell.

In certain embodiments, the medium is a basal culture medium supplemented with the following subtances: glutamine or a stabilized dipeptide of L-alanyl-L-glutamine, IL-34, M-CSF, and Blebbistatin or a derivative thereof. In certain embodiments, the basal culture medium is a basal culture medium supplemented with the following substances: a stabilized dipeptide of L-alanyl-L-glutamine, IL-34, M-CSF, and Blebbistatin or a derivative thereof. In certain embodiments, the medium is composed of the follows: X-VIVO15 + X Glutamax + 25ng/ml IL-34 + 50ng/ml M-CSF + 10 µM Blebbistatin. In some embodiments, the medium is particularly suitable for the expansion culture of a microglial cell.

In certain embodiments, the medium is a basal culture medium supplemented with the following substances: one or more of serum-free substitutes, glutamine or a stabilized dipeptide of L-alanyl-L-glutamine, EGF, FGF2, heparin and Blebbistatin or a derivative thereof. In certain embodiments, the basal culture medium is a basal culture medium supplemented with the following substances: a B27 supplement, a stabilized dipeptide of L-alanyl-L-glutamine, EGF, FGF2, heparin, and Blebbistatin. In certain embodiments, the medium is composed of the follows: DMEM/F12 + 20 µg/mL EGF + 20 µg/mL bFGF + 5 µg/ml heparin + 2% B27 + 1×GlutaMAX + 10 µM Blebbistatin. In some embodiments, the culture medium is particularly suitable for the expansion culture of a neural stem cell.

In certain embodiments, the basal culture medium described in any of the above items is selected from the group consisting of DMEM/F12, Neurobasal, Neural Induction Media, and X-VIVO.

In another aspect, the present application provides a kit, which comprises the aforementioned composition or culture medium, and optionally, further comprises an instruction for use.

In another aspect, the application provides use of a Myosin inhibitor (e.g., Blebbistatin or a derivative thereof (e.g., (S)-(-)-Blebbistatin O-Benzoate)) or a culture medium containing a Myosin inhibitor for maintaining or increasing the number of neural or nerve-related cells in vitro.

In certain embodiments, the neural or nerve-related cells are selected from the group consisting of neural progenitor cells, motor neuron progenitors, cortical neuron progenitors, GABAergic neuron progenitors, serotonin neuron progenitors, midbrain dopamine nerve cell progenitors, or neural stem cells) or glial cells (e.g., astrocytes or microglial cells).

In some embodiments, the medium is the medium described in the second aspect above. It is particularly useful for maintaining or increasing the number of midbrain dopamine neural progenitor cells in vitro.

In certain embodiments, the medium is a basal culture medium supplemented with the following substances: one or more serum-free substitutes, glutamine or a stabilized dipeptide of L-alanyl-L-glutamine, EGF, FGF2, TGFβ1 and Blebbistatin or a derivative thereof. In certain embodiments, the basal culture medium is a basal culture medium supplemented with the following substances: a N2 supplement, a B27 supplement, a stabilized dipeptide of L-alanyl-L-glutamine, EGF, FGF2, TGFβ1 and Blebbistatin. In certain embodiments, the medium is composed of the follows: 48% DMEM/F-12 + 48% Neurobasal + 1% N2 + 2% B27 + 1% GlutaMAX + 10ng/ml EGF + 10ng/ml FGF2 + 10ng/ml TGFβ1 + 10 µM Blebbistatin. It is particularly useful for maintaining or increasing the number of midbrain dopamine neural progenitor cells in vitro. It is particularly useful for maintaining or increasing the number of astrocytes in vitro.

In certain embodiments, the medium is a basal culture medium supplemented with the following substances: one or more serum-free substitute, glutamine or a stabilized dipeptide of L-alanyl-L-glutamine, a SHH signaling pathway inhibitor, and Blebbistatin or a derivative thereof. In certain embodiments, the basal culture medium is a basal culture medium supplemented with the following substances: a N2 supplement, a B27 supplement, a stabilized dipeptide of L-alanyl-L-glutamine, SHH, Purmorphamine, and Blebbistatin. In certain embodiments, the medium is composed of the follows: 48% DMEM/F-12 + 48% Neurobasal + 1% N2 + 2% B27 + 1% GlutaMAX + 100 ng/mL SHH + 1 µM purmorphamine + 10 µM Blebbistatin. It is particularly useful for maintaining or increasing a number of GABAergic neural progenitor cells in vitro.

In certain embodiments, the medium is a basal culture medium supplemented with the following substances: glutamine or a stabilized dipeptide of L-alanyl-L-glutamine, IL-34, M-CSF, and Blebbistatin or a derivative thereof. In certain embodiments, the basal culture medium is a basal culture medium supplemented with the following substances: a stabilized dipeptide of L-alanyl-L-glutamine, IL-34, M-CSF, and Blebbistatin or a derivative thereof. In certain embodiments, the medium is composed of the follows: X-VIVO15 + X Glutamax + 25ng/ml IL-34 + 50ng/ml M-CSF + 10 µM Blebbistatin. It is particularly useful for maintaining or increasing the number of microglial cells in vitro.

In certain embodiments, the medium is a basal culture medium supplemented with the following substances: one or more serum-free substitutes, glutamine or a stabilized dipeptide of L-alanyl-L-glutamine, EGF, FGF2, heparin and Blebbistatin or a derivative thereof. In certain embodiments, the basal culture medium is a basal culture medium supplemented with the following substances: a B27 Supplement, a stabilized dipeptide of L-alanyl-L-glutamine, EGF, FGF2, heparin, and Blebbistatin. In certain embodiments, the medium is composed of the follows: DMEM/F12 + 20 µg/mL EGF + 20 µg/mL bFGF + 5 µg/ml heparin + 2% B27 + 1×GlutaMAX + 10 µM Blebbistatin. It is particularly suitable for maintaining or increasing the number of neural stem cells in vitro.

In certain embodiments, the basal culture medium described in any of the above is selected from the group consisting of DMEM/F12, Neurobasal, Neural Induction Media, and X-VIVO.

In another aspect, the application provides a method for maintaining or increasing the number of cells in vitro, which comprises a step of culturing the cells in the aforementioned medium; or use of the aforementioned medium for maintaining or increasing the number of cells in vitro.

In some embodiments, the method to conduct the culturing is: preparing the cells into a single cell suspension, inoculating the single cell suspension into the culture medium at a density of 2×10⁴/cm² to 6×10⁴/cm² (preferably 4×10⁴/cm²), conducting adherent or suspension culture and passaging the cells once every 5-8 days, wherein the culture conditions of each passage are the same. In certain embodiments, inoculate at a density of 4×10⁴/cm².

In some embodiments, the conditions for the adherent culture are as follows: preparing the cells into a single cell suspension, inoculating the single cell suspension into the culture medium at a density of 2×10⁴/cm² to 6×10⁴/cm², coducting adherent culture and passaging the cells once every 5 to 8 days, wherein the culture conditions for each passage are the same. In certain embodiments, inoculate at a density of 4×10⁴/cm².

In some embodiments, the conditions for the suspension culture are as follows: preparing the cells into a single cell suspension, inoculating the single cell suspension into the culture medium at a density of 2×10⁵/mL to 6×10⁵/mL, conducting suspension culture and passaging the cells once every 5 to 8 days, wherein the culture conditions for each passage are the same. In certain embodiments, inoculate at a density of 4×10⁵/mL.

In certain embodiments, the cells are motor neuron progenitor cells. In certain embodiments, the cells are cortical neuron progenitors. In certain embodiments, the cells are GABAergic neuronal progenitors. In certain embodiments, the cells are serotonin neuronal progenitors. In certain embodiments, the cells are astrocytes. In certain embodiments, the cells are neural stem cells. In certain embodiments, the cells are microglial cells.

In certain embodiments, the expanded cells are genome-identical to the primary cells.

In another aspect, the present application provides a cell or cell population, which is prepared or expanded by any of the methods described above. In another aspect, the present application provides a cell or cell population, wherein >60%, >65%, >70%, >75% >, 80%, >85%, >90%, >95%, > 98% or >99% of the cells specifically express at least one or more of the following markers: PCDHGB1, SOX3, SEMA3D, VGF, NEFL, NTRK2, PCDHGA3, CNTN1, BDNF, STMN1, TNC, FAIM2, CHGB, GAP43, ARPP21, ALCAM, OTP, KCNF1, FOXP1, RTN1, MAPT, IGFBP5, NNAT, CHRNA6, C1QL1, INA, TNR, PHLDA1, ELAVL3, TENM1, NRN1, CRMP1, SCG2, PMP22, and NSG1, preferably, the expression level of the marker is at least about 1.5 times, 2.0 times, 3 times, 5 times, 10 times, 20 times, 30 times, 40 times, 50 times or 100 times higher than that of the primary cells.

In certain embodiments, in the cell or cell population, >60%, >65%, >70%, >75%, >80%, >85%, >90%, >95%, >98% % or >99% of the cells specifically express at least one or more of the following markers: MTND4LP7, AL031777.3, HIST1H2AC, HIST1H1C, HIST1H4H, SYNPO2, LMO2, MGAT2, PDXP, DNAJC6, DNAJC22, ELN, MIR568, MIR1179, MIR6892, MIR7-3HG, ANGPTL1, HSPE1-MOB4, INO80B-WBP1, R3HDML, PMF1-BGLAP, PLP1, AP002748.4, MDFI, RCN3, FST, HSPH1, PCBP1, ASPN, TSPAN8, LINC01866, LEFTY2, GMNC, ATP5MF-PTCD1, CCDC96, ALG14, IL11, A2M, C4B, ITGB4, STC1, TMEM229B, MUC5AC, TAC1, CRABP1, CRABP2, H19, C22orf42, RCAN2, PCSK1, VAT1L, CXCL12, DCN, SSTR1, MAP7D2, PPP2R2C, LRFN5, DIRAS3, CA10, C4A, AP002373.1, AMIGO3, GDA, EDIL3, CFH, TGFBI, CLSTN2, FBLN5, HPCAL4, ADCYAP1R1, NNMT, CD44, SMOC1, CLEC3B, DLX5, LYNX1, SYNC, TCAF1P1, CD9, COL3A1, CAVIN1, LMO4, TCF12, GDE1, GNG3, PEG10, TFPI2, CENPF, CAMK2N1, and MLLT11, preferably, the expression level of the marker is at least about 1.5 times, 2.0 times, 3 times, 5 times, 10 times, 20 times, 30 times, 40 times, 50 times or 100 times higher than that of the primary cells.

In certain embodiments, the method for preparing the cell or cell population comprises the step defined in the method for maintaining or increasing the number of cells in vitro as described in the above aspect.

In certain embodiments, the cell is a neural or nerve-related cell.

In certain embodiments, the cell is selected from the group consisting of motor neuron progenitor, cortical neuron progenitor, GABAergic progenitor, serotonin neuron progenitor, midbrain dopaminergic progenitor, astrocyte, neural stem cell, and microglial cell.

In certain embodiments, the cell is expanded at least about 1.5 times, 2.0 times, 3 times, 5 times, 10 times, 20 times, 30 times, 40 times, 50 times, 100 times, 150 times, 200 times, 500 times, 1000 times, 10000 times or 100000 times from the primary cells.

In another aspect, the cell or cell population provided in the present application is a midbrain dopaminergic progenitor. In certain embodiments, at least 20% (e.g., at least 30%, at least 40%, at least 50%, at least 60%, at least 70%, at least 80%, at least 90%, at least 95%, e.g., 100%) of the cells express at least one marker of midbrain dopamine nerve cell progenitor, for example FOXA2, LMX1A, and OTX2. In certain embodiments, the cells do not express TUJ1. In certain embodiments, the cells express TUJ1. In certain embodiments, in the midbrain dopamine nerve cell progenitors, >60%, >65%, >70%, >75%>, 80%, >85%, >90%, >95%, >98% or >99% of the cells specifically express at least one or more of the following markers: PCDHGB1, SOX3, SEMA3D, VGF, NEFL, NTRK2, PCDHGA3, CNTN1, BDNF, STMN1, TNC, FAIM2, CHGB, GAP43, ARPP21, ALCAM, OTP, KCNF1, FOXP1, RTN1, MAPT, IGFBP5, NNAT, CHRNA6, C1QL1, INA, TNR, PHLDA1, ELAVL3, TENM1, NRN1, CRMP1, SCG2, PMP22, and NSG1, preferably, the expression level of the marker is at least about 1.5 times, 2.0 times, 3 times, 5 times, 10 times, 20 times, 30 times, 40 times, 50 times or 100 times higher than that of the primary cells.

In certain embodiments, in the midbrain dopamine nerve cell progenitors, >60%, >65%, >70%, >75%, >80%, >85%, >90%, >95%, >98% or >99% of the cells specifically express at least one or more of the following markers: MTND4LP7, AL031777.3, HIST1H2AC, HIST1H1C, HIST1H4H, SYNPO2,LMO2, MGAT2, PDXP, DNAJC6, DNAJC22, ELN, MIR568, MIR1179, MIR6892, MIR7-3HG, ANGPTL1, HSPE1-MOB4, INO80B-WBP1, R3HDML, PMF1-BGLAP, PLP1, AP002748.4, MDFI, RCN3, FST, HSPH1, PCBP1, ASPN, TSPAN8, LINC01866, LEFTY2, GMNC, ATP5MF-PTCD1, CCDC96, ALG14, IL11, A2M, C4B, ITGB4, STC1, TMEM229B, MUC5AC, TAC1, CRABP1, CRABP2, H19, C22orf42, RCAN2, PCSK1, VAT1L, CXCL12, DCN, SSTR1, MAP7D2, PPP2R2C, LRFN5, DIRAS3, CA10, C4A, AP002373.1, AMIGO3, GDA, EDIL3, CFH, TGFBI, CLSTN2, FBLN5, HPCAL4, ADCYAP1R1, NNMT, CD44, SMOC1, CLEC3B, DLX5, LYNX1, SYNC, TCAF1P1, CD9, COL3A1, CAVIN1, LMO4, TCF12, GDE1, GNG3, PEG10, TFPI2, CENPF, CAMK2N1, and MLLT11, preferably, the expression level of the marker is at least 1.5 times, 2.0 times, 3 times, 5 times, 10 times, 20 times, 30 times, 40 times, 50 times or 100 times higher than that of the primary cells.

In another aspect, the present application provides a cell or cell population, which is prepared by the following method:
(1) culturing the expanded midbrain dopamine nerve cell progenitors in the first culture medium for 6 days;
(2) culturing the cells obtained in step (1) in the second culture medium for 8 days to obtain the cells or cell population; wherein,

the first culture medium is a basal culture medium added with the following substances: BDNF, GDNF, TGF-β3, FGF8, AA, and Y-27632; preferably, the first culture medium is a basal culture medium supplemented with the following substances: 10-50 ng/ml BDNF, 10-50 ng/ml GDNF, 0.5-5 ng/ml TGF-β3, 50-200 ng/ml FGF8, 0.1-1 mM AA, and 1-30 mM Y-27632; preferably, the first culture medium is a basal culture medium supplemented with the following substances: 20 ng/ml BDNF, 20 ng/ml GDNF, 1 ng/ml TGF-β3, 100 ng/ml FGF8, 0.2 mM AA, and 10 mM Y-27632;
the second culture medium is a basal culture medium supplemented with the following substances: BDNF, GDNF, TGF-β3, FGF8, AA, db-cAMP, and DAPT; preferably, the second culture medium is a basal culture supplemented with the following substances: 10-50 ng/ml BDNF, 10-50 ng/ml GDNF, 0.5-5 ng/ml TGF-β3, 500-200 FGF8, 0.1-1 mM AA, 200-1000 µM db-cAMP, and 5-20 µM DAPT; preferably, the second culture medium is basal culture medium supplemented with: 20 ng/ml BDNF, 20 ng/ml GDNF, 1 ng/ml TGF-β3, 100 FGF8, 0.2 mM AA, 500 µM db-cAMP, and 10 µM DAPT.

In certain embodiments, the basal culture medium is B27.

In certain embodiments, preparing the expanded midbrain dopamine nerve cell progenitors obtained above into a single cell suspension, inoculating the single cell suspension into the first culture medium at a density of 1×10⁴/cm² - 1×10⁶/cm² (for example, 1×10⁴/cm², 5×10⁴/cm², 1×10⁵/cm², 5×10⁴/cm² or 1×10⁶/cm²).

In another aspect, the present application further provides a pharmaceutical composition, which comprises the cell or cell population described in any one of the above aspects.

In another aspect, the present application further provides use of the cell or cell population described in any one of the above aspects in the manufacture of a medicament for treating a nervous system disease (e.g., a disease caused by dopaminergic neuron damage (e.g., Parkinson's disease)).

### Definition of Terms

As used herein, the term "dopamine" refers to a chemical substance produced by dopaminergic neurons that transmits messages to neurons controlling movement and coordination in the brain.

As used herein, the term "neural progenitor cell" refers to an immature nerve cell, including various types of neural progenitor cells, such as motor neuron progenitors, cortical neuron progenitors, GABAergic neuron progenitors, serotonin neuron progenitors, etc.

As used herein, the term "midbrain dopaminergic progenitor" has the same meaning as "midbrain floor plate cell" and refers to a cell located in the midbrain that can develop into dopaminergic neuron, including midbrain dopaminergic neuron that is still in the embryonic development stage. In certain embodiments, the midbrain dopaminergic progenitor expresses one or more of the following cell markers: EN1, FOXA2, LMX1A, OTX2, and the like.

As used herein, the term "dopaminergic neuron" generally refers to a cell that releases dopamine. "Midbrain dopaminergic neuron" refers to a cell in the forebrain that releases dopamine. The cell expresses one or more of the following markers: FOXA2, LMX1A, TH, TUJ1 and the like. As used herein, the term "signaling pathway" refers to a series of reactions for downstream protein to exert its effects that is activated or otherwise affected by a ligand binding to a membrane protein or some other stimulus, for example, SMAD pathway, Wnt pathway. The regulatory factors of the Wnt pathway include β-catenin, GSK3β and the like. For many cell surface or intracellular receptor proteins, the ligand-receptor interaction is not directly linked to a cellular response. The receptor activated by ligand must interact with other intracellular protein before the ligand can exert physiological effects on cellular behavior. Typically, the behavior of several cellular proteins that interact in a chain change with the activation or inhibition of the receptor. The overall cellular changes induced by receptor activation are called as a cellular transduction mechanism or signaling pathway.

As used herein, the term "inhibiting", "blocking" or "hindering" refers to a decrease of activity of a particular signaling pathway in the cells treated with a compound (i.e., an inhibitor).

### Beneficial Effects of the Invention

The present application has achieved at least one of the following technical effects by adjusting the components of the culture medium, adding Myosin II ATPase inhibitor Blebbistatin, and expansion culturing to obtain a variety of neural or nerve-related cells such as midbrain dopaminergic progenitors:
1. The expansion efficiency of neural or nerve-related cells, especially the expansion efficiency of midbrain floor plate cells or midbrain dopaminergic progenitors, is greatly improved, thereby realizing the cell expansion in large scale, and realizing cell line establishment, which are useful in scientific research or commercialization.
2. Based on the improvement of expansion efficiency, in the process of differentiating neural progenitor cells, it is possible to design a technical procedure for expanding intermediate product, thereby obtaining a large number of seed cells from the same batch, and establishing a seed cell bank.
3. In some embodiments, the expanded midbrain dopaminergic progenitors can still express floor plate cell markers FOXA2, LMX1A, OTX2, etc., and can still be used for subsequent cryopreservation.
4. The production costs, especially the cost in the pluripotent stem cell culture phase, can be reduced, so that the preparation process is more controllable.
5. The cells differentiated from the expanded midbrain dopamine nerve cell progenitors specifically express a variety of new markers, and show good therapeutic activity in rat PD models.

### Brief Description of the Drawings

The drawings described here are used to provide a further understanding of the present invention and constitute a part of the application. The schematic examples of the present invention and descriptions thereof are used to explain the present invention and do not constitute improper limitations to the present invention. In the drawings:
Fig. 1 shows the induction and expansion process of floor plate cells derived from human embryonic stem cells.
Fig. 2 shows the cell proliferation curve of each passage in the floor plate expansion media 1 and 2.
Fig. 3 shows the expression of floor plate cell markers LMX1A and FOXA2 of floor plate cells obtained by subculture for 10 passages using the floor plate cell expansion medium 1.
Fig. 4 shows the expression of dopamine neuron markers FOXA2, TH, and TUJ1 of cells obtained by differentiation of floor plate cells cultured for 10 passages using the floor plate cell expansion medium 1.
Fig. 5 shows the expression of cell markers FOXA2 and LMX1A after subculture for 10 passages using the floor plate cell expansion medium 2.
Fig. 6 shows the expression of dopaminergic neuron markers FOXA2, LMX1A, TH, and TUJ1 of cells obtained by differentiation of floor plate cells cultured for 12 passages using the floor plate cell expansion medium 2.
Fig. 7 shows the therapeutic effect of striatal injection of 2.5×10⁵ cells in rat PD models detected by the rotation experiment.
Fig. 8 shows the cell amount of the astrocytes from p1 to p6 under the culture conditions of the experimental group and the control group, +SMI: NPM + 10ng/ml EGF + 10ng/ml FGF2 + 10ng/ml TGFβ1 + 10 µm Blebbistatin; -SMI: NPM + 10ng/ml EGF + 10ng/ml FGF2 + 10ng/ml TGFβ1.
Fig. 9 shows the cell amount of the GABA progenitor cells from p4 to p5 under the culture conditions of the experimental group and the control group, +SMI: NPM + 100 ng/mL SHH + 1 µM purmorphamine + 10 µM Blebbistatin; -SMI: NPM + 100 ng/mL SHH + 1 µM purmorphamine.
Fig. 10 shows the amounts of suspension cells collected from EBs cultured on days 4-11 in microglial differentiation medium (collection 1), CTRL: X-VIVO15 + 1× Glutamax + 25ng/ml IL-34 + 50ng/ml M-CSF; SMI: X-VIVO15 + 1×Glutamax + 25ng/ml IL-34 + 50ng/ml M-CSF + 10 µM Blebbistatin.
Fig. 11 shows the amounts of suspension cells collected from EBs cultured on days 11-18 in microglial differentiation medium (collection 2), CTRL: X-VIVO15 + 1×Glutamax + 25ng/ml IL-34 + 50ng/ml M-CSF; SMI: X-VIVO15 + 1×Glutamax + 25ng/ml IL-34 + 50ng/ml M-CSF + 10 µM Blebbistatin.
Fig. 12 shows the amounts of suspension cells collected from EBs cultured on days 18-25 in microglial differentiation medium (collection 3), CTRL: X-VIVO15 + 1×Glutamax + 25ng/ml IL-34 + 50ng/ml M-CSF; SMI: X-VIVO15 + 1×Glutamax + 25ng/ml IL-34 + 50ng/ml M-CSF + 10 µM Blebbistatin.
Fig. 13 shows the total amount of NSCs at different expansion passages, wherein, A: DMEM/F12 + 20 µg/mL EGF + 20 µg/mL bFGF + 5 µg/ml heparin + 2% B27 + 1×GlutaMAX; B: DMEM/F12 + 20 µg/mL EGF + 20 µg/mL bFGF + 5 µg/ml heparin + 2% B27 + 1× GlutaMAX + 10 µM Blebbistatin.

### Specific Models for Carrying Out the Invention

The technical solutions in the examples of the present invention will be clearly and completely described in conjunction with the accompanying drawings in the examples of the present invention. Obviously, the described examples are only part, not all, examples of the present invention. The following description of at least one exemplary example is merely illustrative in nature and in no way taken as any limitation of the present invention, its application or uses. Based on the examples of the present invention, all other examples obtained by persons of ordinary skill in the art without creative efforts fall within the protection scope of the present invention.

### Example 1. Floor plate cell expansion experiment

### 1. Reagents used in the floor plate cell expansion experiment:

(1) Preparation and working concentration of each factor:
   1) LDN193189 solution: the working concentration was 100 nM. It could be stored at 4°C for two weeks.
   2) SB431542 solution: the working concentration was 10 µM. It could be stored at 4°C for two weeks.
   3) SAG solution: the working concentration was 2 µM. It could be stored at 4°C for two weeks.
   4) SHH (Sonic hedgehog) solution: the working concentration was 100 ng/mL. It could be stored at 4°C for two weeks.
   5) CHIR99021 solution: the working concentration was 0.5-1.0 µM. It could be used once.
   6) Blebbistatin solution: the working concentration was 10 µM. It could be stored at 4°C for two weeks.
(2) Preparation of basal culture medium N2 medium:
   49% CTS^{™} KnockOut^{™} DMEM/F-12 + 49% CTS^{™} Neurobasal + 1% CTS^{™} N2 Supplement +1% CTS- GlutaMAX^{™}-I
(3) Floor plate cell expansion medium 1:
   N2 medium + LDN193189 (100 nM) + SB431542 (10 µM) + SHH (100 ng/mL) + SAG (2 µM) + CHIR99021 (0.5-1.0 µM);
(4) Floor plate cell expansion medium 2:
   N2 medium + LDN193189 (100 nM) + SB431542 (10 µM) + SHH(100 ng/mL) + SAG (2 µM) + CHIR99021 (0.5-1.0 µM) + Blebbistatin(10 µM);

### 2. Reagents used for the differentiation experiment of dopamine neurons from the expanded floor plate cells:

(1) Preparation and working concentration of each factor:
   1) FGF8 solution: the working concentration was 100 ng/mL. It could be stored at 4°C for two weeks.
   2) TGFβ3 solution: the working concentration was 1 ng/mL. It could be stored at 4°C for two weeks.
   3) Ascorbic acid (AA) solution: the working concentration was 0.2 mM. It could be stored at 4°C for two weeks.
   4) Dibutyryl cAMP (db-cAMP) solution: the working concentration was 0.5 mM. It could be stored at 4°C for two weeks.
   5) BDNF solution: the working concentration was 20 ng/mL. It could be stored at 4°C for two weeks.
   6) GDNF solution: the working concentration was 20 ng/mL. It could be stored at 4°C for two weeks.
(2) Preparation of basal culture medium B27 medium:
   97% CTS^{™} Neurobasal + 2% B27-CTS^{™} + 1% CTS- GlutaMAX^{™}-I
(3) Preparation of dopamine neuron differentiation medium 1:
   B27 medium + BDNF (20 ng/mL) + GDNF (20 ng/mL) + TGF-β3 (1 ng/mL) + AA (0.2 mM) + FGF8 (100 ng/mL), each of the factor solution was mixed well and then added in proportion, so as to obtain the differentiation medium 1, which was pipetted repeatedly;
(4) Preparation of dopamine neuron differentiation medium 2:
   B27 medium + BDNF (20 ng/mL) + GDNF (20 ng/mL) + TGF-β3 (1 ng/mL) + AA (0.2 mM) + db-cAMP (500 µM) + DAPT (10 µM), each of the factor solution was mixed well and then added in proportion, so as to obtain the differentiation medium 2, which was pipetted repeatedly.

### 3. method for floor plate cell expansion experiment:

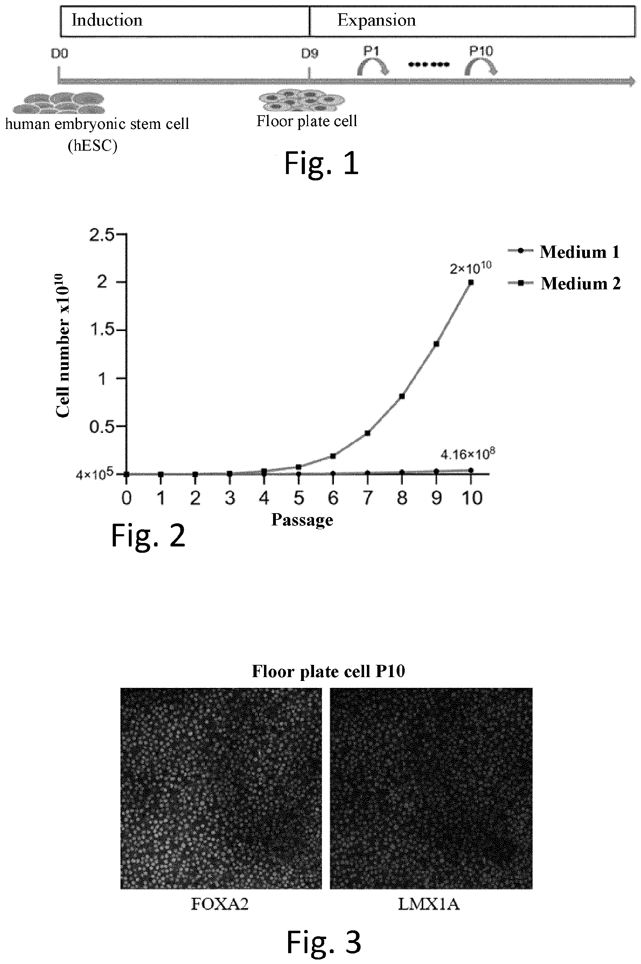

Referring to the method in Figure 1, human embryonic stem cells were subjected to induced differentiation to obtain floor plate cells, which were used for subsequent expansion, cryopreservation and differentiation experiments. The human embryonic stem cells were derived from the National Stem Cell Resource Bank, and commercially available human embryonic stem cell lines could also be used. For the method of inducing floor plate cells from human embryonic stem cells, refer to the prior art, for example doi:10.1038/nprot.2017.078.
(1) Method of obtaining the floor plate cells:
   1) Preparation of Vitronectin matrix: At room temperature, 6 mL of DPBS was pipetted into a 15 mL centrifuge tube, then 60 µL of Vitronectin was pipetted into the above DPBS, mixed well by pipetting with a pipette gun for 10 times to obtain the Vitronectin matrix, which was prepared when being used. 1mL of the prepared matrix was added to each well of a 6-well plate, and incubated at room temperature for 1 hour for use.
   2) Human embryonic stem cells were digested into single cells with TrypLE or accutase, 10 µL of the above suspension was added to 10 µL of trypan blue solution and mixed well, and counting was performed with a countess cell counter; then the Vitronectin matrix was pipetted and discarded, and TrypLE or accutase was used for digestion to obtain single cells, which was inoculated at a density of 2×10⁴/cm² in a differentiation medium containing 10 µM Rock inhibitor (e.g., Y-27632), and culture was conducted in a 37°C, 5% CO₂ incubator. The differentiation medium contained BMP inhibitor and TGF inhibitor, Wnt and SHH activator, and the midbrain floor cells differentiated from the human embryonic stem cells could be obtained after several days.
(2) Floor plate cell expansion experiment:
   1) Preparation of Vitronectin matrix: At room temperature, 6 mL of DPBS was pipetted into a 15 mL centrifuge tube, then 60 µL of Vitronectin was pipetted into the above DPBS, mixed well by pipetting with a pipette gun for 10 times to obtain the Vitronectin matrix, which was prepared when being used. 1ml of the prepared matrix was added to each well of a 6-well plate, and incubated at room temperature for 1 hour for use.
   2) The floor plate cells were digested into single cells with TrypLE or accutase, 10 µL of the above suspension was added to 10 µL of trypan blue solution and mixed well, and counting was performed with a countess cell counter; then the Vitronectin matrix was pipetted and discarded, the cells were re-inoculated at a density of 4×10⁴/cm² by 2D method in the floor plate cell expansion medium 1 and the floor plate cell expansion medium 2 both of which supplemented with 10 µM Rock inhibitor (e.g., Y-27632), and cultured in a 37°C, 5% CO₂ incubator.
   3) The cells were passaged every 5-8 days, and the inoculation density, medium and other conditions of each passage were the same.

At each passage, the total number of cells was calculated using the countess cell counter, and the cell proliferation curve was made.

The proliferation efficiency of the expansion conducted in the floor plate cell expansion media 1 and 2 were shown in Figure 2. Under the treatment of the expansion media 1 and 2, with the increase of cell passage number, the number of cells of Cell 1 and Cell 2 continued to increase, and the proliferation rate gradually decreased. Cell 1 could be expanded up to 10 passages, and Cell 2 could be expanded up to 12 passages; at the same time, the cell number of Cell 2 was 48 times that of Cell 1. The results proved that the expansion medium 2 could greatly increase the expansion ability of the cells.

In the floor plate cell expansion medium 1, with the increase of cell passage number, the number of cells gradually increased, and the proliferation rate gradually decreased. After 10 passages, the cells could be expanded for 1000 times, and the immunofluorescence staining showed that the cells still stably expressed the floor plate cell markers FOXA2 and LMX1A (Figure 3).

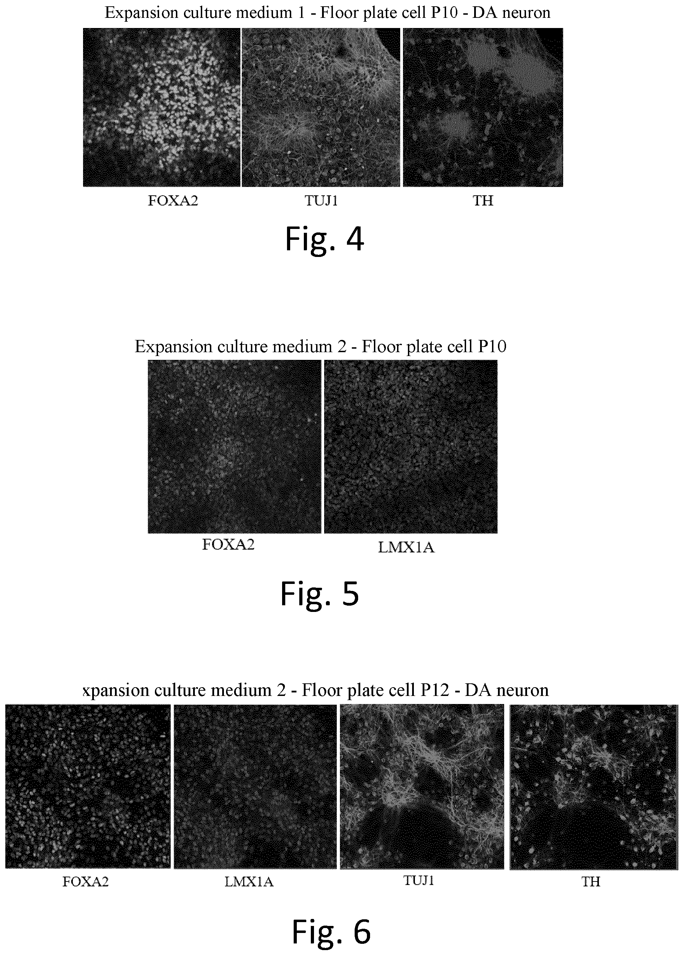

In the floor plate cell expansion medium 2, with the increase of passages, the number of cells gradually increased, and the proliferation rate gradually decreased. After 10 passages, the cells could be expanded for 50,000 times, and the immunofluorescence staining showed that the cells still stably expressed the floor plate cell markers FOXA2 and LMX1A (Figure 5).

### 4. Cryopreservation experiment of floor plate cells after expansion:

1) Programmed cooling boxes and freezing solution were pre-cooled at 4°C for use.
2) The state of the cells was observed under an inverted microscope to ensure that the cells were in good condition and free from contamination.
3) The culture medium was discarded in a biological safety cabinet, and 1mL of DPBS was added to each well of the 6-well plate to wash the cells;
4) DPBS was discarded, 1 mL of Tryple was added to each well, and digestion was performed in a 37°C incubator for 3-4 minutes (the time for digestion by using Tryple could be adjusted depending on the degree of digestion of the cells);
5) Tryple was discarded, 1 mL of basal culture medium N2 medium was added to each well for termination; the cells were gently pipetted into uniformly sized small pieces with a pipette gun, then collected into a centrifuge tube, and centrifuged at 1200 r/min for 3 min at room temperature;
6) The supernatant was discarded, and the pre-cooled freezing solution was added to resuspend the cells (every 1/6 well of cell suspension into one cryopreservation tube, 1mL/tube);
7) The cell suspension was subpackaged into cryopreservation tubes, 1 mL/tube. Then the cryopreservation tubes were placed into a pre-cooled freezer box and stored in a -80°C refrigerator overnight. It was transferred to liquid nitrogen for long-term storage on the next day.

### 5. Experiment of differentiating the expanded floor plate cells into dopamine progenitor cells:

1) Preparation of Vitronectin matrix: At room temperature, 6 mL of DPBS was pipetted into a 15 mL centrifuge tube, then 60 µL of Vitronectin was pipetted into the above DPBS, and mixed well by pipetting with a pipette gun for 10 times to obtain the Vitronectin matrix, which was prepared when being used. 1mL of the prepared matrix was added to each well of a 6-well plate, and incubated at room temperature for 1 hour for later use.
2) The floor plate cells obtained by culturing for 10 passages using the floor plate cell expansion media 1 and 2 were digested with TrypLE or accutase into single cells, which were re-inoculated at a density of 5×10⁵/cm² by the 2D method in the dopamine neuron differentiation medium 1 containing Rock inhibitor (e.g., Y-27632), respectively, after being mixed evenly, they were cultured in a 37°C, 5% CO₂ incubator.
3) After being cultured in the dopamine neuron differentiation medium 1 for 6 days, the cells were further cultured for 8 days by replacing the medium with the dopamine neuron differentiation medium 2.
4) The differentiated dopamine never cells were identified by immunofluorescence, and the results were shown in Figure 4 and Figure 6.

The results showed that the dopamine never cells obtained by further differentiation of Cell 1 obtained by 10 passages of expansion in the floor plate cell expansion medium 1 were identified by immunofluorescence of markers, and the results proved that Cell 1 obtained after 10 passages of expansion still had the ability to differentiate into dopamine nerve cells, and could stably express the markers FOXA2, TH, and TUJ1 of dopamine nerve cells.

The dopamine never cells obtained by further differentiation of Cell 2 obtained by 12 passages of expansion in the floor plate cell expansion medium 2 were identified by immunofluorescence of markers, and the results proved that Cell 2 obtained after 12 passages of expansion still had the ability to differentiate into dopamine never cells, and could stably express the markers FOXA2, LMX1A, TH, TUJ1 of dopamine nerve cells.

Immunofluorescence identification method:
a) Cell inoculation: A clean and sterile circular coverslip was placed in the bottom center of a 24-well cell culture dish, and the cells were inoculated after being coated with vitronectin for 30 minutes;
b) Cell fixation: After the cells grew to an appropriate density, the culture medium was discarded, the cells were washed once with PBS, and fixed with 4% PFA at room temperature for 15-30 min, and then washed 3 times with PBS.
c) Blocking: 320 µL of a mixed solution of 2% BSA and 0.3% Triton was added to each well, and blocking was performed at room temperature for 2 h.
d) Addition of primary antibody: The primary antibody was diluted with a mixed solution of 2% BSA and 0.3% Triton (referring to the instruction manual of the primary antibody for the usage ratio), pipetted and mixed well, the blocking solution was discarded, the diluted primary antibody solution was added, 320µL for each well, and allowed to stand overnight at 4°C after being sealed with parafilm.
e) Addition of secondary antibody: The primary antibody was discarded by pipetting, the cells were washed twice with PBS; 320 µL of the secondary antibody diluted in a mixed solution of 2% BSA and 0.3% Triton was added to each well (referring to the instruction manual of the primary antibody for the usage ratio), and allowed to stand in the dark for 2 hours at room temperature.
f) Staining nuclei: The secondary antibody was discarded, the cells were washed twice with PBS; 320 µL of a diluted Hoechst33342 solution (1 ul of Hoechst33342 in 1 ml of PBS) was added to each well, and the cells were incubated for 15 min.
g) Sealing: The diluted Hoechst33342 solution was discarded by pipetting, the cells were washed once with PBS; then 10 µL of anti-quencher was added to each slide, the glass slide with cells growing thereon was carefully picked up using a syringe needle in cooperation with curved tweezers, and the side with the cells was put down on the glass slide with anti-quencher, avoiding air bubbles; finally, a colorless nail polish was lightly placed on the edge of the cover glass to fix the glass slide, which was then allowed to dry for 5 minutes and put into the slide box.

5) The differentiated cells were subjected to RNA extraction and expression profile sequencing, and the specific method referred to the published literature of Kim et al., Biphasic Activation of WNT Signaling Facilitates the Derivation of Midbrain Dopamine Neurons from hESCs for Translational Use. 2021, Cell Stem Cell 28, 343-355.

The results showed that the expression level of dopamine nerve cells obtained by differentiating the floor plate cells cultured with the expansion medium was more than 10 times that of the unexpanded cells (Tables 1 to 2), and the markers were shown in the table.

**Table 1: Statistics of expression profile results of the expanded cells, part 1**

| | Gene name | Expression level of formula 1 (not expanded) | Expression level of formula 2 (expanded) |
|---|---|---|---|
| Relative mRNA expression level | PCDHGB1 | 3.400765 | 40.19527 |
| | SOX3 | 0.1409564 | 2.220847 |
| | SEMA3D | 0.60748 | 10.76362 |
| | VGF | 14.195183 | 236.2871 |
| | NEFL | 8.330079 | 98.3712 |
| | NTRK2 | 3.228189 | 33.12535 |
| | PCDHGA3 | 0.044837 | 6.693222 |
| | CNTN1 | 1.182307 | 20.33537 |
| | BDNF | 0.480524 | 8.822195 |
| | STMN1 | 0.526314 | 14.26352 |
| | TNC | 24.353844 | 277.0916 |
| | FAIM2 | 1.192175 | 12.36466 |
| | CHGB | 1.089562 | 15.23698 |
| | GAP43 | 17.4865 | 361.2452 |
| | ARPP21 | 0.14378 | 5.665673 |
| | ALCAM | 0.22283 | 7.826541 |
| | OTP | 0 | 5.263871 |
| | KCNF1 | 2.366999 | 26.23453 |
| | FOXP1 | 1.23052 | 15.26389 |
| | RTN1 | 2.35628 | 74.21589 |
| | MAPT | 11.732004 | 161.4916 |
| | IGFBP5 | 0.15872 | 21.33698 |
| | NNAT | 0.36381 | 4.125873 |
| | CHRNA6 | 0 | 3.820327 |
| | C1QL1 | 0.25362 | 3.259631 |
| | INA | 0.25741 | 4.218901 |
| | TNR | 0.109791 | 4.099195 |
| | PHLDA1 | 0.695623 | 7.584162 |
| | ELAVL3 | 0.2513601 | 8.574123 |
| | TENM1 | 0.524152 | 10.55022 |
| | NRN1 | 4.235861 | 71.26374 |
| | CRMP1 | 5.217891 | 59.325743 |
| | SCG2 | 12.978907 | 279.7323 |
| | PMP22 | 0.41231201 | 8.251431 |
| | NSG1 | 12.559338 | 164.1721 |

**Table 2: Statistics of expression profile results of the expanded cells, part 2**

| | Gene name | Expression level of formula 1 (not expanded) | Expression level of formula 2 (expanded) |
|---|---|---|---|
| Relative mRNA expression level | MTND4LP7 | 0 | 76.03553 |
| | AL031777.3 | 0.171489 | 4.468266 |
| | HIST1H2AC | 2.476634 | 48.57276 |
| | HIST1H1C | 1.247798 | 18.05772 |
| | HIST1H4H | 0.117923 | 2.009719 |
| | SYNPO2 | 0.244708 | 3.128491 |
| | LMO2 | 2.664059 | 29.57198 |
| | MGAT2 | 0 | 3.6155988 |
| | PDXP | 1.274254 | 20.21273 |
| | DNAJC6 | 3.984809 | 44.89279 |
| | DNAJC22 | 0.01079 | 7.323679 |
| | ELN | 25.384838 | 271.8633 |
| | MIR568 | 0 | 10.6673 |
| | MIR1179 | 0 | 2.305298 |
| | MIR6892 | 0 | 1.868145 |
| | MIR7-3HG | 0.363221 | 17.53261 |
| | ANGPTL1 | 0.241169 | 5.236946 |
| | HSPE1-MOB4 | 0 | 22.99689 |
| | INO80B-WBP1 | 0 | 15.52343 |
| | R3HDML | 0 | 3.719844 |
| | PMF1-BGLAP | 1.174822 | 12.88243 |
| | PLP1 | 2.91422 | 56.07297 |
| | AP002748.4 | 0 | 4.168712 |
| | MDFI | 2.53376 | 69.14996 |
| | RCN3 | 1.022739 | 19.856729 |
| | FST | 1.768944 | 27.64677 |
| | HSPH1 | 6.78339 | 71.87888 |
| | PCBP1 | 0 | 22.56321 |
| | ASPN | 1.067857 | 17.3869 |
| | TSPAN8 | 0.425994 | 4.261259 |
| | LINC01866 | 0 | 2.114547 |
| | LEFTY2 | 1.675307 | 18.6166 |
| | GMNC | 0.157274 | 4.895082 |
| | ATP5MF-PTCD1 | 0.258379 | 5.889129 |
| | CCDC96 | 0 | 2.845406 |
| | ALG14 | 0.50492 | 13.754033 |
| | IL11 | 0.638582 | 6.99416 |
| | A2M | 0.639516 | 83.66215 |
| | C4B | 0.326981 | 19.03333 |
| | ITGB4 | 0.164464 | 9.896965 |
| | STC1 | 1.107299 | 14.11464 |
| | TMEM229B | 0.21548 | 6.56191 |
| | MUC5AC | 1.282185 | 16.63354 |
| | TAC1 | 7.292313 | 247.3887 |
| | CRABP1 | 0 | 18.25214 |
| | CRABP2 | 0 | 10.23211 |
| | H19 | 0.335919 | 10.90753 |
| | C22orf42 | 0.693401 | 10.93448 |
| | RCAN2 | 2.200737 | 25.01389 |
| | PCSK1 | 9.467299 | 110.4257 |
| | VAT1L | 7.56819 | 92.87996 |
| | CXCL12 | 3.896376 | 39.65608 |
| | DCN | 3.248732 | 37.4826 |
| | SSTR1 | 0.839088 | 8.842989 |
| | MAP7D2 | 0.667324 | 7.378552 |
| | PPP2R2C | 0.842985 | 15.11611 |
| | LRFN5 | 0.364771 | 8.302847 |
| | DIRAS3 | 3.174719 | 42.63766 |
| | CA10 | 0.123762 | 4.743845 |
| | C4A | 0.913978 | 18.53574 |
| | AP002373.1 | 0 | 7.396976 |
| | AMIGO3 | 0.096202 | 6.43058 |
| | GDA | 0.569416 | 8.455555 |
| | EDIL3 | 3.225003 | 46.04006 |
| | CFH | 0.711477 | 29.47776 |
| | TGFBI | 2.282342 | 31.66396 |
| | CLSTN2 | 4.044774 | 41.36876 |
| | FBLN5 | 3.726905 | 45.37458 |
| | HPCAL4 | 3.027599 | 39.77068 |
| | ADCYAP1R1 | 0.562132 | 7.302859 |
| | NNMT | 3.632776 | 71.54955 |
| | CD44 | 2.824466 | 35.9178 |
| | SMOC1 | 10.995736 | 118.502 |
| | CLEC3B | 0.532358 | 8.722571 |
| | DLX5 | 0.015 | 0.51 |
| | LYNX1 | 0 | 4.318749 |
| | SYNC | 1.189385 | 13.70889 |
| | TCAF1P1 | 0 | 8.423484 |
| | CD9 | 1.433858 | 16.51166 |
| | COL3A1 | 61.333252 | 643.8076 |
| | CAVIN 1 | 1.949901 | 28.39877 |
| | LMO4 | 0.836252 | 9.543621 |
| | TCF12 | 0.543213 | 7.642513 |
| | GDE1 | 0.668022 | 8.071253 |
| | GNG3 | 0.522036 | 19.23625 |
| | PEG 10 | 0.985125 | 10.23561 |
| | TFPI2 | 0.549224 | 7.642502 |
| | CENPF | 0.254056 | 38.31252 |
| | CAMK2N1 | 2.301012 | 53.263121 |
| | MLLT11 | 0.782102 | 16.230112 |

### 6. Behavior detection method of PD model (rotation experiment):

Experimental equipment: rotation recorder, counter, weighing scale.
Experimental reagent: apomorphine (sigma, A4393).

The rotation experiment steps were as follows:
a) The animals were weighed;
b) The animals were anesthetized by intraperitoneal injection of 5% chloral hydrate according to body weight;
c) Preparation and injection of apomorphine: The powdered apomorphine was dissolved in normal saline to 1 mg/ml, and injected intraperitoneally at a dose of 0.5 mg/kg;
d) Counting was started 5-10 minutes after the injection of apomorphine or when the model rats started to spin in circles steadily, recorded for 30 minutes, and the average number of turns per minute was calculated.

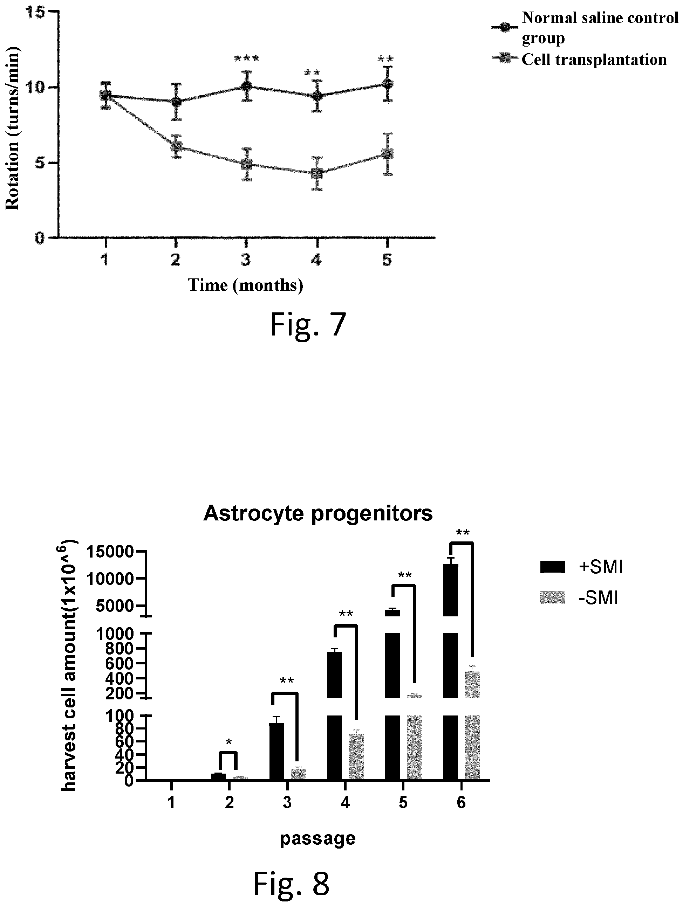

The cell injection steps were as follows:
Experimental equipment: brain stereotaxic instrument, electric injection pump, cranial drill, surgical scissors, tweezers, needle-holding forceps, 5-0 suture with needle, Hamilton 701 syringe, heat preservation pad, ultra-clean workbench, etc.
Experimental reagents: povidone iodine, normal saline, disinfectant alcohol, Baytril, cyclosporine.
   a) Injection of immunosuppressant: 48 hours before transplantation, the immunosuppressant cyclosporine was intraperitoneally injected at a dose of 10 mg/kg, and the injection was performed every day until the end of the experiment;
   b) Preparation of cell suspension: The culture supernatant of the cells to be transplanted was discarded, the cells were washed once with PBS, and then added with tryple to digest for 5-8 minutes; the cell suspension was collected into a centrifuge tube, centrifuged at 1200rpm for 3 minutes, the supernatant was discarded, the cells were resuspended to 1.25×10⁵/µL with cell injection solution;
   c) Injection of cell suspension: 2 µL (2.5×10⁵ cells) of the above cell suspension was injected into the striatum of each animal at the modeling side, and the coordinates of the striatum were: A, +1.0 mm; L, -3.0 mm; V, -6.0 mm, -5.0 mm; 1 needle track, 2 depths, 1 µL was injected for each depth, injection speed was 1 µL/min, and the wound was sutured after injection.
   d) Post-operative care: After the operation, Baytril was subcutaneously injected daily, 0.5mL/200g body weight, for 3 consecutive days. Rotation experiment was performed every month after injection, the change in the number of turns over 5 months was plotted, in which significant functional improvement was observed in the 3^{rd} month (Figure 7).

### Example 2. Expansion experiment of astrocytes

### 1. Differentiation and culture method:

(1) hESCs were cultured and differentiation was conducted until about 80% confluence. On the 0^{th} day, the cells were dissociated with Dispase (1 U/mL), and the bottom cell mass was harvested and plated in an ultra-low adsorption 10 cm culture dish.
(2) From the 1^{st} day, the neural induction was started by replacing with NIM medium supplemented with neural induction molecules 100 nM LDN193189 and 10 µM SB431542. The medium was changed daily until the 7^{th} day.
(3) The cell spheres were spread on a Matrigel-coated 6-well plate on the 7^{th} day.
(4) From the 9^{th} day, the medium was replaced with NPM medium, and 20 ng/mL FGF2 was added until the 13^{th} day.
(5) On the 13^{th} day, Accutase was used for dissociation to obtain single cells, which were re-spread on a Matrigel-coated six-well plate at a density of 1.2×10⁵ cells/cm².
(6) On the 14^{th} day, the medium was replaced with astrocyte induction medium, the NPM medium was added with 10ng/ml EGF + 10ng/ml FGF2 + 10ng/ml TGFβ1, and 10 µm Blebbistatin (1:1000, not added to the control group) was added with at the same time, and the medium was replaced every other day.
(7) After the density confluence of astrocytes reached 100%, they were dissociated into single cells with Accutase and counted, and re-spread on a Matrigel-coated six-well plate at a density of 3.0×10⁵ cells/cm².
(8) The cells were passaged for 5 times in succession, the initial cell density of each passage was 3.0×10⁵ cells/cm², and the proliferation of astrocytes under the addition of Blebbistatin was compared.

### Neural induction medium (NIM)

| H9 hESC | Volume |
|---|---|
| DMEM/F12 | 243.75 ml |
| Neurobasal | 243.75 ml |
| N2 (100X) | 2.5 ml |
| B27 (50X) | 5 ml |
| GlutaMAX (100X) | 5 ml |
| Total | 500ml |

### Neural proliferation medium (NPM):

| H9 hESC | Volume |
|---|---|
| DMEM/F12 | 240 ml |
| Neurobasal | 240 ml |
| N2 (100X) | 5 ml |
| B27 (50X) | 10 ml |
| GlutaMAX (100X) | 5 ml |
| Total | 500ml |

### 2. Experimental results:

From P2 to P6, the expansion ability of astrocytes in the experimental group was significantly improved (Figure 8).

### Example 3. Proliferation of GABA progenitor cells

### 1. Differentiation and culture method:

(1) hESCs were cultured and differentiation was conducted until about 80% confluence. On the 0^{th} day, the cells were dissociated with Dispase (1 U/mL), and the bottom cell mass was harvested and spread on an ultra-low adsorption 10 cm culture dish.
(2) From the 1^{st} day, the neural induction was started by using NIM medium supplemented with 100 nM LDN193189, 10 µM SB431542, and 2 µM XAV939. The medium was changed daily until the 7^{th} day.
(3) The cell spheres were spread on a Matrigel-coated 6-well plate on the 7^{th} day.
(4) From the 9^{th} day, the medium was replaced with NPM medium, and 20 ng/mL FGF2 was added until the 13^{th} day.
(5) On the 13^{th} day, Accutase was used for dissociation to obtain single cells, which were re-spread on a Matrigel-coated 6-well plate at a density of 3.0×10⁵ cells/cm².
(6) On the 14^{th} day, the medium was replaced with GABA cell induction medium, the NPM medium was added with 100 ng/mL SHH and 1 µM purmorphamine, and 10 µm Blebbistatin was added at the same time (not added to the control group), and the medium was replaced every other day.
(7) After the density confluence of GABA progenitor cells reached 100%, they were dissociated into single cells with Accutase and counted, and then re-spread on a Matrigel-coated six-well plate at a density of 3.0×10⁵ cells/cm².
(8) The cells were passaged for 5 times, the initial cell density of each passage was 3.0×10⁵ cells/cm², and the proliferation of GABA progenitor cells with or without Blebbistatin was compared.

### 2. Experimental results:

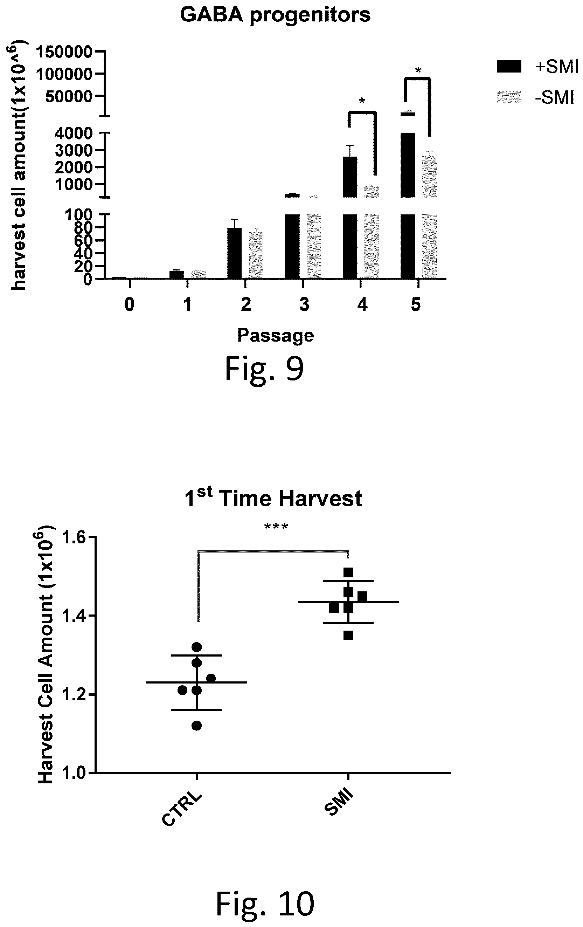

From P4 to P5, the expansion ability of GABA progenitor cells in the experimental group was significantly improved (Figure 9).

### Example 4. Proliferation of microglial progenitors

### 1. Differentiation and culture method:

(1) On the 0^{th} day, ESCs were digested with Accutase into single cells, counted and inoculated into a V-bottom 96-well plate at a concentration of 10,000/well, and cultured statically.
(2) From the 0^{th} to 4^{th} day, the medium was microglial induction medium: DMEM/F12, 20% KOSR, 1×Glutamax, 1X NEAA, 50ng/ml BMP4, 50ng/ml VEGF, 20ng/ml SCF, 10µM Y-27632.
(3) On the 4^{th} day, the suspended EBs were subjected to adherent culture in a 6-well plate, the culture medium was changed on the 4^{th} day and then the static culture was carried out until the 11^{th} day. From the 4^{th} to 11^{th} day, the culture medium was microglial differentiation medium: X-VIVO15, 1×Glutamax, 25ng/ml IL-34, 50ng/ml M-CSF. At this stage, Blebbistatin was added at a concentration of 10 µM.
(4) On the 11^{th} day, the single cells suspended in the medium were collected and counted, and the EBs were continued to be cultured with the microglial differentiation medium, and the suspended cells in the supernatant were collected every 7 days for a total of 3 collections.
(5) Maturation of microglial cells: The supernatant was discarded after centrifugation, the cells were resuspended in a microglial maturation medium and inoculated on Matrigel pre-coated culture plates. The microglial cells matured after being continuously cultured for 14 days. Microglial maturation medium: Advanced F12, 1×Glutamax, 50ng/ml IL-34, 5ng/ml M-CSF.

### 2. Experimental results:

On the 11^{th} day of differentiation, the single cells suspended in the culture medium were collected and counted, and the EBs were continued to be cultured in the microglial differentiation medium, and the suspended cells in the supernatant were collected every 7 days for a total of 3 collections. Number of cells in each time of collection between the experimental group and the control group was compared (Figures 10 to 12). The number of cells in the first two collections of the experimental group increased significantly, which proved that the small molecule could promote the proliferation of microglial progenitors and improve the differentiation efficiency.

### Example 5. Proliferation of neural stem cells

### 1. Differentiation and culture method:

The differentiation method was referred to Tchieu et al., A Modular Platform for Differentiation of Human PSCs into All Major Ectodermal Lineages. 2017, Cell Stem Cell 21, 399-410.

NSCs were cultured with expansion medium after adherence, DMEM/F12 (1×, Gibco) supplemented with 20 µg/mL human (EGF) (R&D), 20 µg/mL (bFGF) (R&D), 5 µg/ml Heparin (Sigma), 2% B27 (Gibco), 1×GlutaMAX, and 10 µM Blebbistatin was added to the experimental group. Half of the culture medium was changed every day.

### 2. Experimental results:

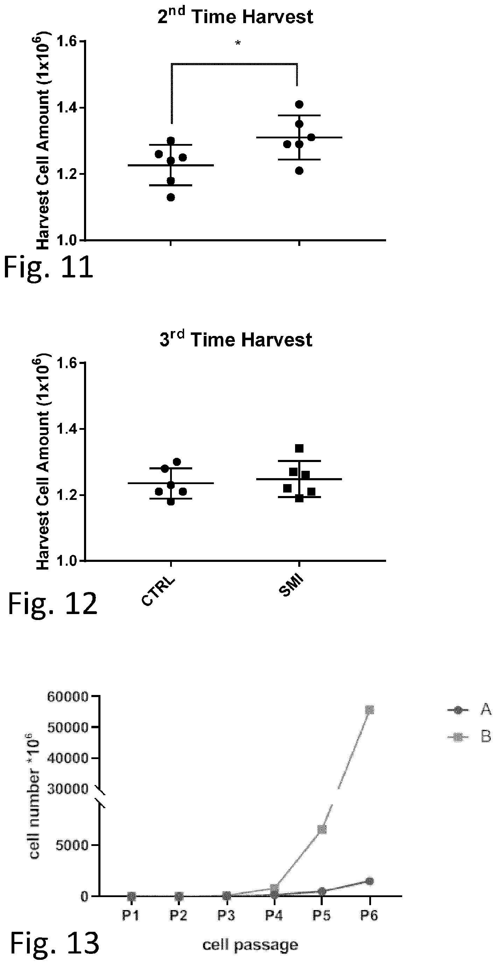

The small molecule significantly promoted the expansion of NSCs from P4 to P6 (see: Figure 13).

Various modifications of the present invention, in addition to those described herein, will be apparent to those skilled in the art from the foregoing description. Such modifications are also intended to fall within the scope of the appended claims. Each reference cited in the present application, including all patents, patent applications, journal articles, books, and any other publications, is hereby incorporated by reference in its entirety.

## Claims

1. A composition, which comprises a SMAD signaling pathway inhibitor, a SHH signaling pathway agonist, a Wnt signaling pathway agonist and a Myosin II ATPase inhibitor, optionally, which further comprises a ROCK inhibitor (e.g., Y-27632);
alternatively, the composition consists of a SMAD signaling pathway inhibitor, a SHH signaling pathway agonist, a Wnt signaling pathway agonist, a Myosin II ATPase inhibitor, and optionally a ROCK inhibitor (e.g., Y-27632).

2. The composition according to claim 1, wherein the Myosin II ATPase inhibitor is selected from the group consisting of Blebbistatin and a derivative thereof (e.g., (S)-(-)-Blebbistatin O-Benzoate).

3. The composition according to claim 1 or 2, wherein the SMAD signaling pathway inhibitor is selected from the group consisting of a BMP inhibitor, a TGFβ/Activin-Nodal inhibitor and a combination thereof;
preferably, the BMP inhibitor is selected from the group consisting of DMH-1, Dorsomorphin, Noggin, LDN193189 and any combination thereof;
preferably, the TGFβ/Activin-Nodal inhibitor is selected from the group consisting of SB431542, SB505124, A83-01 and any combination thereof.

4. The composition according to any one of claims 1-3, wherein the SHH signaling pathway agonist is selected from the group consisting of a SHH protein, a Smoothend agonist and a combination thereof;
preferably, the SHH protein is selected from the group consisting of recombinant SHH and terminal-modified SHH (e.g., SHH C25II);
preferably, the Smoothend agonist is selected from the group consisting of SAG, Hh-Ag1.5, 20α-hydroxycholesterol, Purmorphamine and any combination thereof.

5. The composition according to any one of claims 1-4, wherein the Wnt signaling pathway agonist is selected from the group consisting of a GSK3β inhibitor, Wnt3A, Wnt1 and a combination thereof;
preferably, the GSK3β inhibitor is selected from the group consisting of CHIR99021, GSK3β inhibitor IX (6-bromoindirubin-3'-oxime, BIO), GSK3β inhibitor VII (4-dibromoacetophenone), Indirubin, L803-mts, TWS119, AZD2858, AR-A014418, TDZD-8, LY2090314, 2-D08, IM-12, 1-Azakenpaullone, SB216763 or any combination thereof.

6. The composition according to any one of claims 1-5, which comprises a BMP inhibitor, a TGFβ/Activin-Nodal inhibitor, a SHH protein, a Smoothened agonist, a GSK3β inhibitor, a Myosin II ATPase inhibitor, and optionally a ROCK inhibitor; preferably, it comprises LDN193189, SB431542, a recombinant SHH, SAG, CHIR99021, Blebbistatin, and optionally Y-27632;
alternatively, the composition consists of a BMP inhibitor, a TGFβ/Activin-Nodal inhibitor, a SHH protein, a Smoothened agonist, a GSK3β inhibitor, a Myosin II ATPase inhibitor, and optionally a ROCK inhibitor; preferably, the composition consists of LDN193189, SB431542, a recombinant SHH, SAG, CHIR99021, Blebbistatin and optionally Y-27632.

7. A culture medium, which comprises the composition according to any one of claims 1-6, and a basal culture medium, preferably, the basal culture medium is suitable for the cultivation of a nerve cell.

8. The culture medium according to claim 7, wherein the basal culture medium is a basal culture medium supplemented with the following substances: one or more serum-free substitutes, glutamine or a stabilized dipeptide of L-alanyl-L-glutamine;
preferably, the basal culture medium is a basal culture medium supplemented with the following substances: a N2 supplement and a stabilized dipeptide of L-alanyl-L-glutamine;
preferably, the basal culture medium comprises the following components: 49% CTS^{™} KnockOut^{™} DMEM/F-12 + 49% CTS^{™} Neurobasal + 1% CTS^{™} N2 Supplement + 1% CTS-GlutaMAX^{™}-I.

9. The culture medium according to claim 7 or 8, wherein, in the culture medium, the content of each component of the composition is as follows:
0.05-1 µM BMP inhibitor, 5-20 µM TGFβ/Activin-Nodal inhibitor, 50-200 ng/mL SHH protein, 0.5-3 µM Smoothened agonist, 0.5-1.0 µM GSK3β inhibitor, and 5-20 µM Myosin II ATPase inhibitor;
preferably, 10 µM SB431542, 100 nM LDN193189, 100 ng/mL SHH, 2 µM SAG, 0.5-1.0 µM CHIR99021, and 10 µM Blebbistatin.

10. A culture medium, which comprises the composition according to any one of claims 1-6, and a basal culture medium which is selected from:
(1) the culture medium according to any one of claims 7-9;
(2) a basal culture medium supplemented with the following substances: one or more serum-free substitutes, glutamine or a stabilized dipeptide of L-alanyl-L-glutamine, EGF, FGF2, TGFβ1, and Blebbistatin or a derivative thereof;
preferably, a basal culture medium supplemented with the following substances: a N2 supplement, a B27 supplement, a stabilized dipeptide of L-alanyl-L-glutamine, EGF, FGF2, TGFβ1 and Blebbistatin;
preferably, the culture medium comprises the following components: 48% DMEM/F-12 + 48% Neurobasal + 1% N2 + 2% B27 + 1% GlutaMAX + 10ng/ml EGF + 10ng/ml FGF2 + 10ng/ml TGFβ1 + 10 µm Blebbistatin;
(3) a basal culture medium supplemented with the following substances: one or more serum-free substitutes, glutamine or a stabilized dipeptide of L-alanyl-L-glutamine, a SHH signaling pathway inhibitor, and Blebbistatin or a derivative thereof;
preferably, a basal culutre medium supplemented with the following substances: a N2 supplement, a B27 supplement, a stabilized dipeptide of L-alanyl-L-glutamine, SHH, Purmorphamine and Blebbistatin;
preferably, the culture medium comprises the following components: 48% DMEM/F-12 + 48% Neurobasal + 1% N2 + 2% B27 + 1% GlutaMAX + 100 ng/mL SHH + 1 µM purmorphamine + 10 µm Blebbistatin;
(4) a basal culture medium supplemented with the following substances: glutamine or a stabilized dipeptide of L-alanyl-L-glutamine, IL-34, M-CSF and Blebbistatin or a derivative thereof;
preferably, a basal culture medium supplemented with the following substances: a stabilized dipeptide of L-alanyl-L-glutamine, IL-34, M-CSF, and Blebbistatin or a derivative thereof;
preferably, the culture medium comprises the following components: X-VIVO15 +×Glutamax + 25ng/ml IL-34 + 50ng/ml M-CSF + 10 µM Blebbistatin; and
(5) a basal culture medium supplemented with the following substances: one or more serum-free substitutes, glutamine or a stabilized dipeptide of L-alanyl-L-glutamine, EGF, FGF2, heparin, and Blebbistatin or a derivative thereof;
preferably, a basal culture medium supplemented with the following substances: a B27 supplement, a stabilized dipeptide of L-alanyl-L-glutamine, EGF, FGF2, heparin and Blebbistatin;
preferably, the culture medium comprises the following components: DMEM/F12 + 20 µg/mL EGF + 20 µg/mL bFGF + 5 µg/ml heparin + 2% B27 + 1×GlutaMAX + 10 µM Blebbistatin;
preferably, the basal culture medium described in any of the above items is selected from the group consisting of DMEM/F12, Neurobasal, Neural Induction Media or X-VIVO.

11. A kit, which comprises the composition according to any one of claims 1-6 or the culture medium according to any one of claims 7-10, optionally, which further comprises an instruction for use.

12. A method for maintaining or increasing the number of cells in vitro, which comprises a step of culturing the cells in the culture medium according to any one of claims 7-10;
preferably, the cells are selected from motor neuron progenitor cells, cortical neuron progenitors, GABAergic progenitors, serotonergic progenitors, midbrain dopaminergic progenitors, astrocytes, neural stem cells, or microglial cells;
preferably, the method to conduct the culturing is: preparing the cells into a single cell suspension, inoculating the single cell suspension into the culture medium at a density of 2×10⁴/cm² to 6×10⁴/cm², conducting adherent or suspension culture and passaging the cells once every 5-8 days, wherein the culture conditions of each passage are the same.

13. A cell or cell population, which is prepared by the method according to claim 12.

14. The cell or cell population according to claim 13, wherein the cell is selected from neural or nerve-related cells.

15. The cell or cell population according to claim 13 or 14, the cell is selected from the group consisting of motor neuron progenitor cell, cortical neuron progenitor, GABAergic progenitor, serotonin neuron progenitor, midbrain dopamine never cell progenitor, astrocyte, neural stem cell, or microglial cell.

16. A cell or cell population, or the cell or cell population according to any one of claims 13-15, wherein >60%, >65%, >70%, >75%, >80%, >85%, >90%, >95%, >98% or >99% of the cells specifically express at least one or more of the following markers: PCDHGB1, SOX3, SEMA3D, VGF, NEFL, NTRK2, PCDHGA3, CNTN1, BDNF, STMN1, TNC, FAIM2, CHGB, GAP43, ARPP21, ALCAM, OTP, KCNF1, FOXP1, RTN1, MAPT, IGFBP5, NNAT, CHRNA6, C1QL1, INA, TNR, PHLDA1, ELAVL3, TENM1, NRN1, CRMP1, SCG2, PMP22, and NSG1, preferably, the expression level of the marker is at least about 1.5 times, 2.0 times, 3 times, 5 times, 10 times, 20 times, 30 times, 40 times, 50 times or 100 times higher than that of primary cells.

17. The cell or cell population according to any one of claims 13-16, wherein >60%, >65%, >70%, >75%, >80%, >85%, >90%, >95% %, >98% or >99% of the cells specifically express at least one or more of the following markers: MTND4LP7, AL031777.3, HIST1H2AC, HIST1H1C, HIST1H4H, SYNPO2,LMO2, MGAT2, PDXP, DNAJC6, DNAJC22, ELN, MIR568, MIR1179, MIR6892, MIR7-3HG, ANGPTL1, HSPE1-MOB4, INO80B-WBP1, R3HDML, PMF1-BGLAP, PLP1, AP002748.4, MDFI, RCN3, FST, HSPH1, PCBP1, ASPN, TSPAN8, LINC01866, LEFTY2, GMNC, ATP5MF-PTCD1, CCDC96, ALG14, IL11, A2M, C4B, ITGB4, STC1, TMEM229B, MUC5AC, TAC1, CRABP1, CRABP2, H19, C22orf42, RCAN2, PCSK1, VAT1L, CXCL12, DCN, SSTR1, MAP7D2, PPP2R2C, LRFN5, DIRAS3, CA10, C4A, AP002373.1, AMIGO3, GDA, EDIL3, CFH, TGFBI, CLSTN2, FBLN5, HPCAL4, ADCYAP1R1, NNMT, CD44, SMOC1, CLEC3B, DLX5, LYNX1, SYNC, TCAF1P1, CD9, COL3A1, CAVIN1, LMO4, TCF12, GDE1, GNG3, PEG10, TFPI2, CENPF, CAMK2N1, MLLT11, preferably, the expression level of the marker is at least about 1.5 times, 2.0 times, 3 times, 5 times, 10 times, 20 times, 30 times, 40 times, 50 times or 100 times higher than that of the primary cells.

18. The cell or cell population according to any one of claims 13-15, which is expanded at least about 1.5 times, 2.0 times, 3 times, 5 times, 10 times, 20 times, 30 times, 40 times, 50 times, 100 times, 150 times, 200 times, 500 times, 1000 times, 10000 times or 100000 times from the primary cells.

19. A cell or cell population, wherein the cell is a midbrain dopamine never cell progenitor; preferably, at least 20% (e.g., at least 30%, at least 40%, at least 50%, at least 60%, at least 70%, at least 80%, at least 90%, at least 95%, such as 100%) of the cells express at least a marker of the midbrain dopamine never cell progenitor, for example FOXA2, LMX1A, and OTX2.

20. The cell or cell population according to claim 19, wherein the cell does not express TUJ1.

21. The cell or cell population according to claim 19 or 20, wherein the cell has any one or more of the characteristics as defined in any one of claims 13-18.

22. The cell or cell population according to claim 19, wherein the cell expresses TUJ1.

23. The cell or cell population according to claim 22, wherein the cell has one or more of the characteristics as defined in any one of claims 13-18.

24. A pharmaceutical composition, which comprises the cell or cell population according to any one of claims 13-23.

25. Use of the cell or cell population according to any one of claims 13-23 in the manufacture of a medicament for treating a nervous system disease (e.g., a disease caused by dopamine nerve cell damage (e.g., Parkinson's disease)).

26. A method for treating a nervous system disease (e.g., a disease caused by dopamine nerve cell damage (e.g., Parkinson's disease)), which comprises administering a subject in need thereof an effective amount of the cell or cell population according to any one of claims 13-23.
